# EUROPÄISCHE PATENTSCHRIFT

(11) **EP 1 633 691 B1**
(45) Veröffentlichungstag und Bekanntmachung des Hinweises auf die Patenterteilung: **11.04.2007**
(21) Anmeldenummer: 04739502.5
(22) Anmeldetag: 02.06.2004
(51) Int. Cl.: C07C 37/60, C07C 39/15

(54) **VERFAHREN ZUR HERSTELLUNG VON 2,2'-DIHYDROXY-BIPHENYLEN**
METHOD FOR PRODUCING 2,2'-DIHYDROXY-BIPHENYLS
PROCEDE DE PREPARATION DE 2,2'-DIHYDROXY-BIPHENYLS

(30) Priorität: 04.06.2003 DE 10325490
(43) Veröffentlichungstag der Anmeldung: 15.03.2006
(73) Patentinhaber: BASF Aktiengesellschaft, 67056 Ludwigshafen (DE)
(72) Erfinder: FLORES, Miguel Angel, E-28300 Aranjuez (ES); BARTSCH, Michael, 67433 Neustadt (DE); BAUMANN, Robert, 68161 Mannheim (DE); HADERLEIN, Gerd, 67269 Gründstadt (DE); JUNGKAMP, Tim, B-2950 Kapellen (BE); SCHEIDEL, Jens, 69493 Hirschberg (DE); LUYKEN, Hermann, 67069 Ludwigshafen (DE); SIEGEL, Wolfgang, 67117 Limburgerhof (DE); MEYER, Christa, 67229 Gerolsheim (DE); WIDMAIER, Ralf, 68167 Mannheim (DE)
(86) Internationale Anmeldenummer: PCT/EP2004/005915
(87) Internationale Veröffentlichungsnummer: WO 2004/108642

(56) Entgegenhaltungen:
- WO-A-96/23074
- US-A- 6 077 979

## Beschreibung

Die vorlegende Erfindung betrifft ein Verfahren zur Herstellung eines 2,2'-Dihydroxy-biphenyls durch oxidative Kupplung von zwei Phenolmolekülen, die in einer o-Stellung ein Wasserstoffatom aufweisen, mittels eines Peroxids in Gegenwart von Wasser bei einer Temperatur im Bereich von 0°C bis 100°C.

2,2'-Dihydroxy-biphenyle, wie 2,2'-Dihydroxy-3,3',5,5'-tetramethyl-biphenyl, können bekanntermaßen für die Herstellung von Bidentat-Phosphit-, Bidentat-Phosphonit- und Bidentat-Phosphinit-Liganden eingesetzt werden.

Bekanntermaßen können 2,2'-Dihydroxy-biphenyle durch oxidative Kupplung von zwei Phenolmolekülen, die in einer o-Stellung ein Wasserstoffatom aufweisen, mittels eines Peroxids in Gegenwart von Wasser bei einer Temperatur im Bereich von 0°C bis 100°C hergestellt werden.

So beschreibt US 6,077,979 die Herstellung von 2,2'-Dihydroxy-3,3',5,5'-tetramethyl-biphenyl durch oxidative Kupplung von zwei Molekülen 2,4-Dimethylphenol mittels Persulfat und katalytische aktiven Mengen an Eisen oder einer Eisen-Verbindung in Gegenwart von Wasser bei einer Temperatur im Bereich von 0°C bis 100°C.

WO 96/23074 beschreibt einen biokatalytischen Prozess zur Herstellung von Tetraalkylbiphenolen.

Problematisch bei dieser Reaktion ist, dass sich bei der Umsetzung eine gummiartige, klebrige Masse bildet, die zu einer Belegung des Reaktors und der Reaktoreinbauten, wie Rührer, führt. Dies hat insbesondere bei der Durchführung in technischem Maßstab zur Folge, dass ein Wärmeaustausch und eine Durchmischung des Reaktionsgemischs kaum noch möglich ist, ein vollständiger Umsatz der Edukte nicht erzielt werden kann und zudem die Aufarbeitung des erhaltenen Produktgemischs außerordentlich erschwert wird.

Der vorliegenden Erfindung lag die Aufgabe zugrunde, ein Verfahren bereitzustellen, dass die Herstellung eines 2,2'-Dihydroxy-biphenyls durch oxidative Kupplung von zwei Phenolmolekülen, die in einer o-Stellung ein Wasserstoffatom aufweisen, mittels eines Peroxids in Gegenwart von Wasser bei einer Temperatur im Bereich von 0°C bis 100°C und die Aufarbeitung des erhaltenen Produktgemischs auf technisch einfache und wirtschaftliche Weise unter Vermeidung der genannten Nachteile ermöglicht.

Demgemäß wurde ein Verfahren zur Herstellung eines 2,2'-Dihydroxy-biphenyls durch oxidative Kupplung von zwei Phenolmolekülen, die in einer o-Stellung ein Wasserstoffatom aufweisen, mittels eines Peroxids in Gegenwart von Wasser bei einer Temperatur im Bereich von 0°C bis 100°C, dadurch gekennzeichnet, dass man die Herstellung in Gegenwart eines in Wasser unlöslichen Polymers, das
a) 0,1 bis 99,9 Gew.-% mindestens eines Vinylheterocyclus
b) 0,1 bis 10 Gew.-% mindestens einer difunktionellen Vernetzerkomponente
c) 0 bis 99,8 Gew.-% Styrol oder mindestens eines einfach ungesättigten Styrolderivats oder deren Gemische,
wobei sich die Gew.-%-Angaben der Einzelkomponenten a), b) und c) zu 100 % addieren,
durchführt,
gefunden.

Phenole, die in einer o-Stellung ein Wasserstoffatom aufweisen, sind allgemein bekannt und kommerziell verfügbar.

Die Herstellung einer der bevorzugten Ausgangsverbindungen 2,4-Dimethylphenol ist bekannt und die Verbindung kommerziell verfügbar.

In einer weiteren bevorzugten Ausführungsform kann man als Phenol, das in einer o-Stellung ein Wasserstoffatom aufweist, Phenol (Hydroxybenzol) einsetzen.

In einer anderen bevorzugten Ausführungsform kann man als Phenol, das in einer o-Stellung ein Wasserstoffatom aufweist, 2,3,5-Trimethylphenol einsetzen.

In einer weiteren bevorzugten Ausführungsform kann man als Phenol ein 2-AlkylPhenol, vorzugsweise ein C₁-C₄-Alkylphenol, wie 2-Methyl-phenol, 2-Ethyl-phenol, 2-n-Propyl-phenol, 2-i-Propyl-phenol, 2-n-Butyl-phenol, 2-t-Butyl-phenol, 2-i-Butyl-phenol, insbesondere 2-Methyl-phenol oder 2-t-Butyl-phenol, einsetzen.

In einer weiteren bevorzugten Ausführungsform kann man als Phenol ein 2,4-Dialkyl-Phenol, vorzugsweise der Art 2-R1-4-R2-Phenol, in der R1, R2 unabhängig voneinander Methyl, Ethyl, n-Propyl, i-Propyl, n-Butyl, t-Butyl oder i-Butyl bedeuten, wie 2,4-Dimethyl-phenol, 2-Methyl-4-ethyl-phenol, 2-Methyl-4-n-propyl-phenol, 2-Methyl-4-i-propyl-phenol, 2-Methyl-4-n-butyl-phenol, 2-Methyl-4-t-butyl-phenol, 2-Methyl-4-i-butyl-phenol, 2-Ethyl-4-methyl-phenol, 2,4-Diethyl-phenol, 2-Ethyl-4-n-propyl-phenol, 2-Ethyl-4-i-propyl-phenol, 2-Ethyl-4-n-butyl-phenol, 2-Ethyl-4-t-butyl-phenol, 2-Ethyl-4-i-butyl-phenol, 2-n-Propyl-4-methyl-phenol, 2-n-Propyl-4-ethyl-phenol, 2,4-Di-n-propyl-phenol, 2-n-Propyl-4-i-propyl-phenol, 2-n-Propyl-4-n-butyl-phenol, 2-n-Propyl-4-t-butyl-phenol, 2-n-Propyl-4-i-butyl-phenol, 2-i-Propyl-4-methyl-phenol, 2-i-Propyl-4-ethyl-phenol, 2-i-Propyl-4-n-propyl-phenol, 2,4-Di-i-propyl-phenol, 2-i-Propyl-4-n-butyl-phenol, 2-i-Propyl-4-t-butyl-phenol, 2-i-Propyl-4-i-butyl-phenol, 2-n-Butyl-4-methyl-phenol, 2-n-Butyl-4-ethyl-phenol, 2-n-Butyl-4-n-propyl-phenol, 2-n-Butyl-4-i-propyl-phenol, 2,4-Di-n-butyl-phenol, 2-n-Butyl-4-t-butyl-phenol, 2-n-Butyl-4-i-butyl-phenol, 2-t-Butyl-4-methyl-phenol, 2-t-Butyl-4-ethyl-phenol, 2-t-Butyl-4-n-propyl-phenol, 2-t-Butyl-4-i-propyl-phenol, 2-t-Butyl-4-n-butyl-phenol, 2,4-Di-t-Butyl-phenol, 2-t-Butyl-4-i-butyl-phenol, 2-i-Butyl-4.-methyl-phenol, 2-i-Butyl-4-ethyl-phenol, 2-i-Butyl-4-n-propyl-phenol, 2-i-Butyl-4-i-propyl-phenol, 2-i-Butyl-4-n-butyl-phenol, 2-i-Butyl-4-t-butyl-phenol, 2,4-Dii-butyl-phenol, insbesondere 2,4-Di-methyl-phenol oder 2,4-Di-t-butyl-phenol, insbesondere bevorzugt 2,4-Dimethyl-phenol, einsetzen.

In einer weiteren bevorzugten Ausführungsform kann man als Phenol ein 2,5-Dialkyl-Phenol, vorzugsweise der Art 2-R1-5-R2-Phenol, in der R1, R2 unabhängig voneinander Methyl, Ethyl, n-Propyl, i-Propyl, n-Butyl, t-Butyl oder i-Butyl bedeuten, wie 2,5-Dimethyl-phenol, 2-Methyl-5-ethyl-phenol, 2-Methyl-5-n-propyl-phenol, 2-Methyl-5-i-propyl-phenol, 2-Methyl-5-n-butyl-phenol, 2-Methyl-5-t-butyl-phenol, 2-Methyl-5-i-butyl-phenol, 2-Ethyl-5-methyl-phenol, 2,5-Diethyl-phenol, 2-Ethyl-5-n-propyl-phenol, 2-Ethyl-5-i-propyl-phenol, 2-Ethyl-5-n-butyl-phenol, 2-Ethyl-5-t-butyl-phenol, 2-Ethyl-5-i-butyl-phenol, 2-n-Propyl-5-methyl-phenol, 2-n-Propyl-5-ethyl-phenol, 2,5-Di-n-propyl-phenol, 2-n-Propyl-5-i-propyl-phenol, 2-n-Propyl-5-n-butyl-phenol, 2-n-Propyl-5-t-butyl-phenol, 2-n-Propyl-5-i-butyl-phenol, 2-i-Propyl-5-methyl-phenol, 2-i-Propyl-5-ethyl-phenol, 2-i-Propyl-5-n-propyl-phenol, 2,5-Di-i-propyl-phenol, 2-i-Propyl-5-n-butyl-phenol, 2-i-Propyl-5-t-butyl-phenol, 2-i-Propyl-5-i-butyl-phenol, 2-n-Butyl-5-methyl-phenol, 2-n-Butyl-5-ethyl-phenol, 2-n-Butyl-5-n-propyl-phenol, 2-n-Butyl-5-i-propyl-phenol, 2,5-Di-n-butyl-phenol, 2-n-Butyl-5-t-butyl-phenol, 2-n-Butyl-5-i-butyl-phenol, 2-t-Butyl-5-methyl-phenol, 2-t-Butyl-5-ethyl-phenol, 2-t-Butyl-5-n-propyl-phenol, 2-t-Butyl-5-i-propyl-phenol, 2-t-Butyl-5-n-butyl-phenol, 2,5-Di-t-Butyl-phenol, 2-t-Butyl-5-i-butyl-phenol, 2-i-Butyl-5-methyl-phenol, 2-i-Butyl-5-ethyl-phenol, 2-i-Butyl-5-n-propyl-phenol, 2-i-Butyl-5-i-propyl-phenol, 2-i-Butyl-5-n-butyl-phenol, 2-i-Butyl-5-t-butyl-phenol, 2,5-Dii-butyl-phenol, insbesondere 2-Isopropyl-5-methyl-phenol, einsetzen.

In einer weiteren bevorzugten Ausführungsform kann man als Phenol ein 3,5-Dialkyl-Phenol, vorzugsweise der Art 3-R1-5-R2-Phenol, in der R1, R2 unabhängig voneinander Methyl, Ethyl, n-Propyl, i-Propyl, n-Butyl, t-Butyl oder i-Butyl bedeuten, wie 3,5-Dimethyl-phenol, 2-Methyl-5-ethyl-phenol, 3-Methyl-5-n-propyl-phenol, 3-Methyl-5-i-propyl-phenol, 3-Methyl-5-n-butyl-phenol, 3-Methyl-5-t-butyl-phenol, 3-Methyl-5-i-butyl-phenol, 3-Ethyl-5-methyl-phenol, 3,5-Diethyl-phenol, 3-Ethyl-5-n-propyl-phenol, 3-Ethyl-5-i-propyl-phenol, 3-Ethyl-5-n-butyl-phenol, 3-Ethyl-5-t-butyl-phenol, 3-Ethyl-5-i-butyl-phenol, 3-n-Propyl-5-methyl-phenol, 3-n-Propyl-5-ethyl-phenol, 3,5-Di-n-propyl-phenol, 3-n-Propyl-5-i-propyl-phenol, 3-n-Propyl-5-n-butyl-phenol, 3-n-Propyl-5-t-butyl-phenol, 3-n-Propyl-5-i-butyl-phenol, 3-i-Propyl-5-methyl-phenol, 3-i-Propyl-5-ethyl-phenol, 3-i-Propyl-5-n-propyl-phenol, 3,5-Di-i-propyl-phenol, 3-i-Propyl-5-n-butyl-phenol, 3-i-Propyl-5-t-butyl-phenol, 3-i-Propyl-5-i-butyl-phenol, 3-n-Butyl-5-methyl-phenol, 3-n-Butyl-5-ethyl-phenol, 3-n-Butyl-5-n-propyl-phenol, 3-n-Butyl-5-i-propyl-phenol, 3,5-Di-n-butyl-phenol, 3-n-Butyl-5-t-butyl-phenol, 3-n-Butyl-5-i-butyl-phenol, 3-t-Butyl-5-methyl-phenol, 3-t-Butyl-5-ethyl-phenol, 3-t-Butyl-5-n-propyl-phenol, 3-t-Butyl-5-i-propyl-phenol, 3-t-Butyl-5-n-butyl-phenol, 3,5-Di-t-Butyl-phenol, 3-t-Butyl-5-i-butyl-phenol, 3-i-Butyl-5-methyl-phenol, 3-i-Butyl-5-ethyl-phenol, 3-i-Butyl-5-n-propyl-phenol, 3-i-Butyl-5-i-propyl-phenol, 3-i-Butyl-5-n-butyl-phenol, 3-i-Butyl-5-t-butyl-phenol, 3,5-Dii-butyl-phenol, insbesondere 3,5-Di-methyl-phenol oder 3,5-Di-t-butyl-phenol, einsetzen.

In einer weiteren bevorzugten Ausführungsform kann man als Phenol ein 2,3,5-Trialkyl-Phenol, vorzugsweise der Art 2-R1-3-R2-5-R3-Phenol, in der R1, R2, R3 unabhängig voneinander Methyl, Ethyl, n-Propyl, i-Propyl, n-Butyl, t-Butyl oder i-Butyl bedeuten, insbesondere 2,3,5-Trimethylphenol, einsetzen.

Besonders bevorzugt kommt der Einsatz von 2,4-Dimethyl-phenol als Phenol in Betracht.
Erfindungsgemäß wird als 2,2'-Dihydroxybiphenyl 2,2'-Dihydroxy-3,3',5,5'-Tetramethyl-biphenyl erhalten.

Es können ebenfalls Gemische von Phenolen, insbesondere der genannten Phenole eingesetzt werden.

Vorteilhaft kommt der Einsatz nur eines Phenols in Betracht.

Das erfindungsgemäße Verfahren ist besonders vorteilhaft im Falle solcher Phenole, die bei der gewählten Umsetzungstemperatur flüssig vorliegen und deren durch Kupplung entsprechend erhaltenes 2,2'-Dihydroxybiphenyl bei dieser Temperatur fest vorliegt.
Besonders bevorzugt kommt der Einsatz von 2,4-Dimethyl-phenol als Phenol in Betracht bei einer Umsetzung im Bereich von 20-50°C unter Erhalt von 2,2'-Dihydroxy-3,3',5,5'-Tetramethyl-biphenyl als 2,2'-Dihydroxybiphenyl.

Setzt man Phenole ein, die bei der gewählten Umsetzungstemperatur fest vorliegen, so hat es sich als vorteilhaft erwiesen, das Phenol in einem flüssigen Verdünnungsmittel, vorzugsweise einem organischen flüssigen Verdünnungsmittel, insbesondere Hexan, aufzulösen, zu der Mischung ein unlösliches Polymer, enthaltend
a) 0,1 bis 99,9 Gew.-% mindestens eines Vinylheterocyclus
b) 0,1 bis 10 Gew.-% mindestens einer difunktionellen Vernetzerkomponente
c) 0 bis 99,8 Gew.-% Styrol oder mindestens eines einfach ungesättigten Styrolderivats oder deren Gemische,
wobei sich die Gew.-%-Angaben der Einzelkomponenten a), b) und c) zu 100 % addieren,
zugibt,
das flüssige Verdünnungsmittel entfernt, vorzugsweise durch Destillation, und das erhaltene Produkt zu der Reaktionsmischung zur Herstellung eines 2,2'-Dihydroxy-biphenyls gibt.

Erfindungsgemäß führt man die Umsetzung bei einer Temperatur im Bereich von 0 bis 100°C durch.
In einer vorteilhaften Ausführungsform kommen Temperaturen von höchstens 50°C in Betracht.
In einer vorteilhaften Ausführungsform kommen Temperaturen von mindestens 15°C in Betracht.

Erfindungsgemäß führt man die Kupplungsreaktion mittels eines Peroxids durch.
Als Peroxid kommt ein organisches, vorzugsweise anorganisches Peroxid in Betracht, wobei ein Peroxid oder Gemische von Peroxiden eingesetzt werden können.

Als organisches Peroxid kann man vorteilhaft eine Verbindung der Formel Formel (tert.-Butyl)-O-O-R mit R = H, tert.-Butyl oder deren Gemische, insbesondere Di-tert.-butyl-peroxid, einsetzen.
Verfahren zur Herstellung solcher Peroxide sind an sich bekannt und die Peroxide kommerziell verfügbar.

Als anorganisches Peroxid kann vorzugsweise ein Persulfat-Anion eingesetzt werden, insbesondere in Form eines Salzes, welches in Wasser eine mindestens geringe Löslichkeit aufweist. Bevorzugte Persulfate sind solche, die als Kation ein Ammonium-, Natrium-, Kalium-Kation oder deren Gemische enthalten.
Als anorganisches Peroxid kann Wasserstoffperoxid, insbesondere zusammen mit Persulfat-Anion, eingesetzt werden, vorzugsweise in einer wässrigen Lösung, enthaltend zwischen 3 und 55 Gew.-% Wasserstoffperoxid.

Zur Erzielung eines hohen Umsatzes ist theoretisch die Zugabe von 0,5 mol für die Kupplung reaktiven Peroxid-Einheiten pro mol Phenol erforderlich. Die Zugabe einer Menge von 0,5 bis 1,2 mol, insbesondere 0,6 bis 0,8 mol, für die Kupplung reaktiven Peroxid-Einheiten pro mol Phenol hat sich als vorteilhaft erwiesen.

In einer bevorzugten Ausführungsform kann man die erfindungsgemäße Umsetzung in Gegenwart von als Katalysator geeigneten Mengen eines Metalls, vorzugsweise Eisen, Cobalt, Kupfer, Mangan, Titan, Silber, Palladium, besonders bevorzugt Eisen, Kupfer, insbesondere Eisen, oder einer Verbindung eines solchen Metalls durchführen. Im Sinne der vorliegenden Erfindung wird unter einem Metall ein Metall oder ein Gemisch mehrer Metalle und unter einer Verbindung eines solchen Metalls eine oder mehrere Verbindungen eines oder mehrerer Metalle verstanden.

Setzt man Eisen ein, so kann dies vorteilhaft in Form einer Eisen enthaltenden Legierung erfolgen, aus der in der Reaktionsmischung Eisen herausgelöst wird. Setzt man Eisen in Form einer Eisen-Verbindung ein, so kann das Eisen in 2- oder 3-wertiger Form vorliegen. Hierbei kann das Eisen als organische, vorzugsweise anorganische Verbindung, insbesondere als Sulfat oder Chlorid eingesetzt werden.
Die Menge an eingesetztem Eisen ist an sich nicht kritisch. Vorzugsweise kann man 0,01 mol Eisen/mol Phenol bis 0,10 mol Eisen/mol Phenol, insbesondere 0,02 bis 0,05 mol Eisen/pro mol Phenol, einsetzen.

Erfindungsgemäß führt man die Umsetzung in Gegenwart eines in Wasser unlöslichen Polymers, enthaltend
a) 0,1 bis 99,9 Gew.-% mindestens eines Vinylheterocyclus
b) 0,1 bis 10 Gew.-% mindestens einer difunktionellen Vernetzerkomponente
c) 0 bis 99,8 Gew.-% Styrol oder mindestens eines einfach ungesättigten Styrolderivats oder deren Gemische,
wobei sich die Gew.%-Angaben der Einzelkomponenten a), b) und c) zu 100 % addieren,
durch.

Dabei kann man als Vinylheterocyclus vorzugsweise mindestens ein N-Vinyllactam oder mindestens ein N-Vinylamin oder deren Gemische einsetzen.
Als N-Vinyllactam kommt vorteilhaft N-Vinylpyrrolidon, N-Vinylpiperidon, N-Vinylcaprolactam oder deren Gemische in Betracht.
Als N-Vinylamin kann man vorzugsweise Vinylimidazol, N-Vinyl-2-methylimidazol, N-Vinyl-4-methylimidazol oder deren Gemische einsetzen.
In einer besonders bevorzugten Ausführungsform kann man ein in Wasser unlösliches Polymer, enthaltend
a) 0,1 bis 99,9 Gew.-% N-Vinylpyrrolidon als Vinylheterocyclus
b) 0,1 bis 10 Gew.-% mindestens einer difunktionellen Vernetzerkomponente
c) 0 bis 99,8 Gew.-% Styrol oder mindestens eines einfach ungesättigten Styrolderivats oder deren Gemische,
wobei sich die Gew.%-Angaben der Einzelkomponenten a), b) und c) zu 100 % addieren,
einsetzen.

Solche Polymere und deren Herstellung sind an sich bekannt, beispielsweise aus DE-A-199 20 944 , EP-A-0177812, EP-A-0088964, EP-A-0351363, WO 02/32544, EP-A-1219349, EP-A-1234608, PCT-Anmeldung Nr. PCT/EP03/03439, EP-Anmeldung Nr. 02027072.4.

Bevorzugt geeignete Polymere sind kommerziell verfügbar, beispielsweise unter der Marke Divergan^{®} RS oder Divergan^{®} F von BASF Aktiengesellschaft, Ludwigshafen/Rhein, Deutschland.

Die erfindungsgemäße Kupplung kann man im wesentlichen nach an sich bekannten Verfahren durchführen, beispielsweise wie in US 6,077,979 beschrieben.

Dabei kann man das in Wasser unlösliche Polymere vor, während oder nach der Kupplungsreaktion zu der Reaktionsmischung zugeben, wobei eine Zugabe vor der Umsetzung bevorzugt ist.

Die Menge an Phenol, bezogen auf Polymer, kann in Abhängigkeit von der Durchführung der Kupplungsreaktion in unterschiedlichen Bereichen optimal gewählt werden. Gibt man das umzusetzende Phenol vor der Kupplungsreaktion vollständig zu, so hat sich eine Menge an Phenol, bezogen auf 1 g Polymer, im Bereich von 10 bis 100 mmol als vorteilhaft herausgestellt. Gibt man das Phenol während der Kupplungsreaktion allmählich zu der Reaktionsmischung, so hat sich eine Menge an Phenol, bezogen auf 1 g Polymer, im Bereich von 50 bis 150 mmol als vorteilhaft erwiesen.

Nach der erfindungsgemäßen Umsetzung kann das Produkt von der Reaktionsmischung nach an sich bekannten Verfahren, wie durch Filtration oder Extraktion, abgetrennt werden.

So kann beispielsweise die Reaktionsmischung filtriert werden unter Erhalt eines Filtrats und eines Filterkuchens. Aus dem Filterkuchen kann dann das Produkt vorteilhaft durch Extraktion, vorzugsweise mit einem aliphatischen, insbesondere aromatischen Kohlenwasserstoff, wie Benzol, o.Xylol, m-Xylol, p-Xylol, insbesondere Toluol, abgetrennt werden. Aus der erhaltenen Lösung kann dann das Produkt erhalten werden, beispielsweise durch Kristallisation oder Abdestillieren des Extraktionsmittels.

Weiterhin kann das Produkt aus der Reaktionsmischung extrahiert werden, vorzugsweise mit einem aliphatischen, insbesondere aromatischen Kohlenwasserstoff, wie Benzol, o.Xylol, m-Xylol, p-Xylol, insbesondere Toluol. Aus der erhaltenen Lösung kann dann das Produkt erhalten werden, beispielsweise durch Kristallisation oder Abdestillieren des Extraktionsmittels.

### Beispiele

### Beispiele 1-5

In einem 250 ml Kolben, der ein Thermometer trug und mit einem Teflonblattrührer ausgestattet war (350 Umdrehungen/Min), wurden 0,7 g (2,5 mmol) FeSO₄.7H₂O in 75 ml Wasser vorgelegt und 1,3 g Divergan RS (Fa. BASF Aktiengesellschaft, Ludwigshafen/Rhein, Deutschland) zugegeben. Die Suspension wurde auf eine Temperatur gemäß Tabelle 1 aufgeheizt. Anschließend wurden 6,1 g (50 mmol) 2,4-Dimethylphenol zudosiert.

Die Suspension bestand aus Flocken und ließ sich gut rühren.
Dann wurde ein bestimmtes Volumen (siehe Tabelle 1) von einer 19 Gew. % wässerige Natriumpersulfat Lösung (1 mol Äquivalent/ 180min) mittels einer Pumpe (Metrohm-Dosimat 665) innerhalb der in Tabelle 1 genannten Zeit zudosiert. Nach Zulaufende wurde die Mischung noch 4 Std. gerührt. Die Suspension wurde auf Raumtemperatur abgekühlt und 199 ml Toluol zugegeben. Die dreiphasige Suspension wurde über eine Nutsche filtriert. Die organische Phase wurde mit Wasser gewaschen und eingeengt. Der Rückstand wurde mittels Gas-Chromatographie analysiert und Umsatz, Selektivität und Ausbeute mittels eines Standards bestimmt (siehe Tabelle 1).

Vergleichsbeispiel (gemäß US 6077979)

In einem 1000 ml Kolben, der ein Thermometer trug und mit einem Teflonblattrührer ausgestattet war (350 Umdrehungen/Min), wurden 4,18 g (15 mmol) FeSO₄.7H₂O in 450 ml Wasser vorgelegt. Anschließend wurden 36,8 g (300 mmol) 2,4-Dimethylphenol zudosiert.
Zu der zweiphasigen Mischung wurde eine wässerige Natriumpersulfat Lösung (71,5 g Na₂S₂O₈ in 300 ml Wasser) mittels einer Pumpe (Metrohm-Dosimat 665) innerhalb von 300 min. bei Raumtemperatur zudosiert.
Nach einiger Zeit entstand einen schmierigen Feststoff, der teilweise an der Reaktorwand und dem Rührer klebte und eine effektive Durchmischung des Reaktionsgemischs nicht ermöglichte. Nach Zulaufende wurde die Mischung noch 3 Tage gerührt. Innerhalb dieser Zeit wurde der schmierige Niederschlag hart.

**Tabelle 1**

| Beispiel | Zulaufzeit [min] | Peroxid/Phenol [mol/mol] | Temperatur [°C] | Umsatz [%] | Selektivität [%] | Ausbeute [%] |
|---|---|---|---|---|---|---|
| 1 | 180 | 1,0 | 60 | 100 | 53 | 53 |
| 2 | 180 | 1,0 | 40 | 94 | 75 | 71 |
| 3 | 90 | 0,5 | 60 | 91 | 74 | 67 |
| 4 | 90 | 0,5 | 50 | 90 | 77 | 69 |
| 5 | 90 | 0,5 | 40 | 89 | 77 | 69 |

## Patentansprüche

1. Verfahren zur Herstellung eines 2,2'-Dihydroxy-biphenyls durch oxidative Kupplung von zwei Phenolmolekülen, die in einer o-Stellung ein Wasserstoffatom aufweisen, mittels eines Peroxids in Gegenwart von Wasser bei einer Temperatur im Bereich von 0°C bis 100°C, **dadurch gekennzeichnet, dass** man die Herstellung in Gegenwart eines in Wasser unlöslichen Polymers, enthaltend
a) 0,1 bis 99,9 Gew.-% mindestens eines Vinylheterocyclus
b) 0,1 bis 10 Gew.% mindestens einer difunktionellen Vernetzerkomponente
c) 0 bis 99,8 Gew.-% Styrol oder mindestens eines einfach ungesättigten Styrolderivats oder deren Gemische,
wobei sich die Gew.-%-Angaben der Einzelkomponenten a), b) und c) zu 100 % addieren,
durchführt.

2. Verfahren nach Anspruch 1, wobei man das Verfahren bei einer Temperatur im Bereich von 15°C bis 50°C durchführt.

3. Verfahren nach Anspruch 1 oder 2, wobei man als Peroxid ein anorganisches Peroxid einsetzt.

4. Verfahren nach Anspruch 1 oder 2, wobei man als Peroxid ein Persulfat-Anion einsetzt.

5. Verfahren nach Anspruch 4, wobei man das Persulfat-Anion in Gegenwart eines Ammonium-, Natrium- oder Kalium-Kations einsetzt.

6. Verfahren nach den Ansprüchen 1 bis 5, wobei man die Umsetzung in Gegenwart von als Katalysator geeigneten Mengen an Eisen oder einer Eisen-Verbindung durchführt.

7. Verfahren nach den Ansprüchen 1 bis 6, wobei man als Phenol 2,4-Dimethylphenol einsetzt unter Erhalt von 2,2'-Dihydroxy-3,3',5,5'-Tetramethyl-biphenyl als 2,2'-Dihydroxy-biphenyl.

8. Verfahren nach den Ansprüchen 1 bis 7, wobei man als Vinylheterocyclus mindestens ein N-Vinyllactam oder mindestens ein N-Vinylamin oder deren Gemische einsetzt.

9. Verfahren nach Anspruch 8, wobei man als N-Vinyllactam N-Vinylpyrrolidon, N-Vinylpiperidon, N-Vinylcaprolactam oder deren Gemische einsetzt.

10. Verfahren nach Anspruch 8, wobei man als N-Vinylamin N-Vinylimidazol, N-Vinyl-2-methylimidazol, N-Vinyl-4-methylimidazol oder deren Gemische einsetzt.

11. Verfahren nach den Ansprüchen 1 bis 9, wobei man ein in Wasser unlösliches Polymer, enthaltend
a) 0,1 bis 99,9 Gew.-% N-Vinylpyrrolidon als Vinylheterocyclus
b) 0,1 bis 10 Gew.-% mindestens einer difunktionellen Vernetzerkomponente
c) 0 bis 99,8 Gew.-% Styrol oder mindestens eines einfach ungesättigten Styrolderivats oder deren Gemische,
wobei sich die Gew.%-Angaben der Einzelkomponenten a), b) und c) zu 100 % addieren,
einsetzt.

## Claims

1. A process for preparing a 2,2'-dihydroxybiphenyl by oxidatively coupling two phenol molecules which have a hydrogen atom in an o-position by means of a peroxide in the presence of water at a temperature in the range from 0°C to 100°C, which comprises carrying out the preparation in the presence of a water-insoluble polymer, comprising
a) from 0.1 to 99.9% by weight of at least one vinyl heterocycle
b) from 0.1 to 10% by weight of at least one difunctional crosslinker component
c) from 0 to 99.8% by weight of styrene or of at least one monounsaturated styrene derivative or a mixture thereof,
the percentages by weight of the individual components a), b) and c) adding up to 100%.

2. The process according to claim 1, which is carried out at a temperature in the range from 15°C to 50°C.

3. The process according to claim 1 or 2, wherein the peroxide used is an inorganic peroxide.

4. The process according to claim 1 or 2, wherein the peroxide used is a persulfate anion.

5. The process according to claim 4, wherein the persulfate anion is used in the presence of an ammonium, sodium or potassium cation.

6. The process according to any of claims 1 to 5, wherein the reaction is carried out in the presence of suitable catalytic amounts of iron or of an iron compound.

7. The process according to any of claims 1 to 6, wherein the phenol used is 2,4-dimethylphenol to obtain 2,2'-dihydroxy-3,3',5,5'-tetramethylbiphenyl as the 2,2'-dihydroxybiphenyl.

8. The process according to any of claims 1 to 7, wherein the vinyl heterocycle used is at least one N-vinyllactam or at least one N-vinylamine or a mixture thereof.

9. The process according to claim 8, wherein the N-vinyllactam used is N-vinylpyrrolidone, N-vinylpiperidone, N-vinylcaprolactam or a mixture thereof.

10. The process according to claim 8, wherein the N-vinylamine used is N-vinylimidazole, N-vinyl-2-methylimidazole, N-vinyl-4-methylimidazole or a mixture thereof.

11. The process according to any of claims 1 to 9, wherein a water-insoluble polymer is used which comprises
a) from 0.1 to 99.9% by weight of N-vinylpyrrolidone as the vinyl heterocycle
b) from 0.1 to 10% by weight of at least one difunctional crosslinker component
c) from 0 to 99.8% by weight of styrene or of at least one monounsaturated styrene derivative or a mixture thereof,
the percentages by weight of the individual components a), b) and c) adding up to 100%.

## Revendications

1. Procédé pour la préparation d'un 2,2'-dihydroxy-biphényle par couplage oxydatif de deux molécules de phénol qui, dans une position 0, présentent un atome d'hydrogène, au moyen d'un peroxyde en présence d'eau à une température se situant dans une plage de 0 °C à 100 °C, **caractérisé en ce que** l'on exécute la préparation en présence d'un polymère insoluble dans l'eau, contenant :
a) 0,1 à 99,9% en poids d'au moins un hétérocycle vinylique
b) 0,1 à 10% en poids d'au moins un composant de réticulant difonctionnel
c) 0 à 99,8% en poids de styrène ou d'au moins un dérivé du styrène simplement insaturé ou leurs mélanges,
les indications de % en poids des différents composants a), b) et c) s'additionnant à 100%.

2. Procédé selon la revendication 1, dans lequel on exécute le procédé à une température dans une plage comprise entre 15 °C et 50 °C.

3. Procédé selon la revendication 1 ou 2, dans lequel on met en oeuvre un peroxyde inorganique en tant que peroxyde.

4. Procédé selon la revendication 1 ou 2, dans lequel on met en oeuvre un anion de persulfate en tant que peroxyde.

5. Procédé selon la revendication 4, dans lequel on met en oeuvre l'anion de persulfate en présence d'un cation d'ammonium, de sodium ou de potassium.

6. Procédé selon les revendications 1 à 5, dans lequel on exécute la conversion en présence de quantités de fer ou d'un composé du fer appropriés comme catalyseur.

7. Procédé selon les revendications 1 à 6, dans lequel on met en oeuvre comme phénol du 2,4-diméthylphénol avec maintien du 2,2'-dihydroxy-3,3',5,5'-tétraméthylbiphényle comme 2,2'-dihydroxy-biphényle.

8. Procédé selon les revendications 1 à 7, dans lequel on met en oeuvre comme hétérocycle vinylique au moins un N-vinyllactame ou au moins une N-vinylamine ou leurs mélanges.

9. Procédé selon la revendication 8, dans lequel on met en oeuvre comme N-vinyllactame, de la N-vinylpyrrolidone, de la N-vinylpipéridone, du N-vinylcaprolactame ou leurs mélanges.

10. Procédé selon la revendication 8, dans lequel on met en oeuvre comme N-vinylamine du N-vinylimidazol, du N-vinyl-2-méthylimidazol, du N-vinyl-4-méthylimidazol ou leurs mélanges.

11. Procédé selon les revendications 1 à 9, dans lequel on met en oeuvre un polymère insoluble dans l'eau contenant :
a) 0,1 à 99,9% en poids de N-vinylpyrrolidone comme hétérocycle vinylique
b) 0,1 à 10% en poids d'au moins un composant de réticulant difonctionnel
c) 0 à 99,8% en poids de styrène ou d' au moins un dérivé de styrène simplement insaturé ou leurs mélanges,
les indications de % en poids des différents composants a), b) et c) s'additionnant à 100%.
